# EUROPÄISCHE PATENTSCHRIFT

(11) **EP 2 243 418 B1**
(45) Veröffentlichungstag und Bekanntmachung des Hinweises auf die Patenterteilung: **29.02.2012**
(21) Anmeldenummer: 09158261.9
(22) Anmeldetag: 20.04.2009
(51) Int. Cl.: A61B 1/12, A61L 2/18

(54) **Vorrichtung und Verfahren zum Behandeln und Analysieren von Kanälen in Instrumenten, insbesondere in Endoskopen**
Device and method for handling and analysing channels in instruments, in particular endoscopes
Dispositif et procédé de traitement et d'analyse de canaux dans des instruments, notamment des endoscopes

(43) Veröffentlichungstag der Anmeldung: 27.10.2010
(73) Patentinhaber: Belimed AG, 6300 Zug (CH)
(72) Erfinder: Medici, Antonio, 6280 Hochdorf (CH)
(74) Vertreter: Hepp Wenger Ryffel AG

(56) Entgegenhaltungen:
- EP-A- 1 502 538
- DE-A1- 4 314 364
- US-A1- 2004 134 520
- US-A1- 2005 209 507

## Beschreibung

Die Erfindung betrifft eine Vorrichtung und ein Verfahren zum Behandeln und Analysieren von Kanälen in Instrumenten, insbesondere in Endoskopen gemäss dem Oberbegriff der unabhängigen Ansprüche. Dabei handelt es sich um eine maschinelle Aufbereitung der Instrumente, bei denen es sich um humanmedizinische oder veterenärmedizinische Endoskope handeln kann, aber im Einzelfall auch um andere Instrumente für industrielle oder wissenschaftliche Zwecke mit Kanälen, die nach einer bestimmten Einsatzzeit des Instruments gereinigt werden müssen. Unter dem Aufbereitungsprozess wird bei Endoskopen üblicherweise ein Prozess verstanden, der aus einer Abfolge von Spülen, Reinigen, Desinfizieren und Trocknen besteht. Um Sicherzustellen, dass in den Kanälen keine Verunreinigungen zurückgeblieben sind, muss der Zustand der Kanäle während des Aufbereitungsprozesses analysiert werden, wobei die Durchlässigkeit der Kanäle ein Mass für deren Reinheit bildet.

Es sind bereits zahlreiche gattungsmässig vergleichbare Verfahren und Vorrichtungen bekannt. Gemäss der DE 103 52 198 wird ein bestimmtes Volumen einer Behandlungsflüssigkeit mit einem vorbestimmten Druck durch einen Endoskopkanal gedrückt, wobei die Zeitdauer für diesen Vorgang gemessen wird. Die JP 2001 299 697 und die EP 1 502 538 beschreiben eine Vorrichtung zum Waschen und Desinfizieren von Endoskopen, bei der die Durchflussrate in jedem der jeweiligen Endoskopkanäle bestimmbar ist, wobei ein Flusssensor verwendet wird. Eine weitere Vorrichtung zum Reinigen und Testen von Endoskopen ist in der US 5, 279, 799 beschrieben. Die US 2004//0134520 betrifft ein Verfahren zum Feststellen von Verstopfungen in den Durchgängen eines medizinischen Instruments. Ebenso betrifft die US 2005/0209507 ein Endoskop-Waschsystem, eine Endoskop-Waschvorrichtung und ein Endoskop.

Ein Problem bei den bekannten Verfahren und Vorrichtungen besteht insbesondere darin, dass Rückstände eines vorangegangenen Prozessschrittes in den nächsten Prozessschritt verschleppt werden können, sodass beispielsweise Reinigungsmittel in den nachfolgenden Desinfektionsprozess gelangt. Dieses Problem entsteht dadurch, dass bei den bekannten Vorrichtungen das Leitungssystem aus flexiblen Schläuchen besteht, in denen sich Taschen bilden können, die nach dem Abschluss eines Prozessschrittes nicht mehr entleerbar sind. Weitere Nachteile der bekannten Systeme bestehen darin, dass die zum Teil unterschiedlichen und/oder verzweigten Kanäle eines Endoskops nicht einzeln behandelt und auch einzeln analysiert werden können. Mangelhaft im bekannten Stand der Technik sind teilweise auch die eigentlichen Messverfahren, die nur auf bestimmte Behandlungsfluide reagieren. Schliesslich kann ein unbemerktes Leck im System auftreten, so dass nicht genügend Behandlungsfluid zum Instrument gelangt.

Es ist daher eine Aufgabe der Erfindung, eine Vorrichtung der eingangs genannten Art bereit zu stellen, mit deren Hilfe eine absolut zuverlässige und automatisierbare Behandlung und Analyse der Instrumente möglich ist. Dabei soll insbesondere das Verschleppen von Behandlungsmitteln eines Prozessschritts in einen nachfolgenden Prozessschritt verhindert werden. Ausserdem soll die Messung der Strömung zuverlässig und unter stets gleichen Betriebsbedingungen erfolgen. Die Vorrichtung soll ausserdem die Behandlung und Analyse einer möglichst grossen Anzahl Einzelkanäle im gleichen Prozess ermöglichen.

Diese Aufgabe wird mit einer Vorrichtung gelöst, welche die Merkmale im Anspruch 1 aufweist. Das Leitungssystem weist dabei eine Messstrecke auf und die Messeinrichtung verfügt über wenigstens einen Strömungssensor, der in der Messstrecke angeordnet ist. Alle Behandlungsschritte sind über das Leitungssystem und insbesondere über die Messstrecke mit dem Strömungssensor ausführbar. Dies hat den grossen Vorteil, dass der Durchfluss sämtlicher Behandlungsfluide, gleichgültig ob flüssig oder gasförmig und gleichgültig in welcher Reihenfolge messbar ist und dass ausserdem das ganze Leitungssystem und die Sensorik den gleichen Prozessen unterworfen werden kann, wie das Instrument selbst. Der Fluidanschluss ist mit wenigstens einer Behandlungsfluidquelle verbindbar und vorzugsweise ist ein Fördermittel zum Durchleiten des Behandlungsfluids durch das Leitungssystem und durch den angeschlossenen Kanal vorhanden. Beim Fördermittel kann es sich beispielsweise um eine Pumpe handeln. Denkbar ist aber auch eine andere Druckmittelquelle, wie z.B. ein unter Luftdruck gesetzter Tank usw.

Erfindungsgemäss sind in der Messstrecke zwei Strömungssensoren nacheinander angeordnet, mit denen die Strömung redundant messbar ist. Damit wird ein besonders hoher Grad an Sicherheit erreicht, weil während des Prozesses auch eine Kontrolle der Sensoren stattfindet. Messen die beiden Sensoren ungleiche Werte, kann dies auf eine Störung an einem der Sensoren hinweisen.

Vorteilhaft erfolgt die Messung der Strömung dabei an einer Messstrecke, die unmittelbar unter der Ebene verläuft, auf welcher der Kanalanschluss liegt, wobei das gesamte Leitungssystem und insbesondere die Messstrecke zur horizontalen Ebene geneigt ist und vorzugsweise senkrecht dazu ausgerichtet ist. Durch die aufsteigende Anordnung des Leitungssystems kann es vollständig von Behandlungsflüssigkeit geleert werden, ohne dass sich Taschen mit Rückständen bilden können. Die Entleerung ist dabei bereits unter Schwerkrafteinwirkung bei deaktiviertem Fördermittel möglich. Alternativ ist aber auch ein Absaugen möglich.

Beim Strömungssensor handelt es sich besonders vorteilhaft um einen thermoelektrischen Sensor, der die Strömung ohne bewegliche Teile ermittelt. Thermoelektrische Strömungssensoren sind an sich bekannt und gebräuchlich. Ein thermischer Flusssensor ist beispielsweise in der DE 43 14 364 beschrieben. Das Messprinzip beruht darauf, dass das durchströmende Fluid eine Abkühlung bewirkt, wobei mit Hilfe eines Heizelements eine konstante Temperatur aufrecht erhalten wird. Der dem Heizelement zugeführte Strom ist ein Mass für den Durchfluss des Fluids.

Zwischen dem Strömungssensor und dem Kanalanschluss ist vorteilhaft ein Einlassventil angeordnet, welches das ganze Leitungssystem vor Beginn des Aufbereitungsprozesses nach aussen absperrt. Diese Absperrung erfolgt aus Sicherheitsgründen und um eine Rückkontamination des Leitungssystems zu verhindern.

Weitere Vorteile können erreicht werden, wenn mehrere Leitungssysteme zu einer Messeinheit zusammengefasst sind, wobei jedes Leitungssystem zu einem Leitungsanschluss führt und wobei vorzugsweise alle Leitungsanschlüsse auf einem gemeinsamen Anschlusskopf angeordnet sind. Auf diese Weise können mehrkanalige Instrumente und/oder auch mehrere Instrumente gleichzeitig behandelt werden, wobei jeder einzelne Kanal über ein autarkes Messsystem verfügt. Am Anschlusskopf sind alle Leitungsanschlüsse zusammengefasst, was die Handhabung erheblich erleichtert und was ausserdem aus Platzgründen vorteilhaft ist.

Alle Leitungssysteme können über eine gemeinsame Verteilkammer mit dem Behandlungsfluid speisbar sein. Bei identisch ausgebildeten Leitungssystemen herrscht so ersichtlicherweise in jedem Leitungssystem der gleiche Betriebsdruck. Die gemeinsame Verteilkammer erleichtert auch das gleichzeitige und schnelle Entleeren des Leitungssystems bzw. das Wechseln von einem Behandlungsfluid zum Nächsten.

Aus konstruktiven Gründen ist es besonders vorteilhaft, wenn alle Leitungssysteme, vorzugsweise wenigstens vier, vorzugsweise kreisförmig oder sternförmig um eine zentrale, vorzugsweise vertikale Achse angeordnet sind. In einer besonders vorteilhaften Anordnung sind acht Leitungssysteme kreisförmig angeordnet. Bei dieser Anordnung sind insbesondere die Strömungssensoren und allfällige weitere Komponenten wie z.B. Elektronik, Ventile usw. von aussen gut zugänglich.

Der vorstehend erwähnet Anschlusskopf kann bei der beschriebenen Anordnung der Leitungssysteme mittels einer Hubvorrichtung aus einer Ruhestellung, von der Messeinheit weg in eine vorzugsweise höher gelegene Betriebsstellung anhebbar sein, in der die Leitungsanschlüsse an einen, einem Aufnahmekorb für die Instrumente zugeordneten Anschlussadapter andockbar sein. Damit kann der Aufnahmekorb mit dem Anschlussadapter ausserhalb des Geräts bzw. ausserhalb der Behandlungskammer bestückt werden, wobei in einer jeweils vorgeschriebenen Ordnung die einzelnen Kanäle des Instruments an den Adapter angeschlossen werden. Nach dem Einfahren des Aufnahmekorbs in die Gerätekammer erfolgt automatisch der Anschluss der Kanalsysteme. Selbstverständlich wäre es denkbar, auf die genau gleiche Art und Weise auch nur ein einziges Kanalsystem an einen Anschlussadapter anzudocken bzw. einzelne Gruppen von Kanalsystemen individuell andockbar auszugestalten.

Die Verbindung zwischen der Messeinheit und dem Anschlusskopf erfolgt vorteilhaft über Steigrohre, die teleskopartig in der Messeinheit verschiebbar sind. Auf diese Weise kann die gesamte Messeinheit fest unter der Behandlungskammer im Gerät montiert werden, wobei sich lediglich die Steigrohre mit dem Anschlusskopf zwischen der Ruhestellung und der Betriebsstellung bewegen.

Das Leitungssystem besteht vorzugsweise ausschliesslich aus starren Rohrleitungen, um das Bilden von Taschen zu vermeiden. Die starren Rohrleitungen haben ausserdem den Vorteil, dass sie sich beispielsweise unter der Einwirkung von Wärme oder Chemikalien nicht verformen können, sodass keine ungewollte Querschnittsveränderung eintritt. Unter dem Begriff Rohrleitung wird grundsätzlich jede Leitungsführung verstanden, also beispielsweise auch eine Bohrung in einem Materialblock.

In verfahrensmässiger Hinsicht wird die Aufgabe mit Hilfe eines Verfahrens mit den Merkmalen im Anspruch 11 gelöst. Die Messung erfolgt dabei mittels eines Strömungssensors an einer Messstrecke und alle Behandlungsschritte werden über das Leitungssystem und insbesondere über die Messstrecke mit dem Strömungssensor ausgeführt. Auf diese Weise ist sichergestellt, dass auch die Messstrecke mit der Sensorik permanent den gleichen Behandlungsschritten unterworfen wird wie das zu behandelnde Instrument. Dies betrifft insbesondere auch die gleiche Behandlungstemperatur und insbesondere auch wenigstens eine chemische und/oder thermische Desinfektion. Das Leitungssystem unterliegt somit permanent einer Selbstreinigung und die Keimzahlreduktion im Leitungssystem ist die Gleiche wie in dem zu behandelnden Instrument.

Dabei wird die Strömung an jeder Messstrecke mittels zweier Strömungssensoren redundant gemessen, wobei die beiden Messungen unabhängig ausgewertet und miteinander verglichen werden. Dabei kann eine Vergleichseinrichtung eingesetzt werden, die auf einen bestimmten Toleranzwert eingestellt ist, sodass beim Überschreiten dieses Toleranzwerts der Prozess automatisch unterbrochen und/oder ein Warnsignal ausgelöst wird.

Die Förderung des Behandlungsfluids bezogen auf eine horizontale Ebene von unten nach oben in der Messstrecke und vorzugsweise im gesamten Leitungssystem erlaubt eine zuverlässige Messung unter stets gleichen hydrostatischen Bedingungen und ausserdem eine vollständige Entleerung des Leitungssystems und insbesondere der Messstrecke.

Das Behandlungsfluid wird dabei vorzugsweise gleichzeitig unter dem gleichen Druck durch mehrere Leitungssysteme gefördert, von denen jedes zu einem Kanalanschluss führt, wobei die Strömung an jedem Leitungssystem gemessen wird.

Es ist ausserdem für eine präzise Analyse der Kanäle besonders vorteilhaft, wenn verschiedene Behandlungsfluide nacheinander durch das gleiche Leitungssystem gefördert werden und wenn die Strömung einzelner oder aller Behandlungsfluide gemessen wird. Die vorzugsweise eingesetzten thermoelektrischen Sensoren ermöglichen dabei nicht nur eine Strömungsmessung an beliebigen Flüssigkeiten, sondern auch an Gasen. So ist es beispielsweise denkbar, den Zustand der Kanäle auch beim Durchleiten von Trocknungsluft zu ermitteln.

Weitere Vorteile und Einzelmerkmale der Erfindung ergeben sich aus der nachfolgenden Beschreibung eines Ausführungsbeispiels und aus den Zeichnungen. Es zeigen:
- Figur 1: eine perspektivische Darstellung eines Aufbereitungs- geräts mit geöffneter Türe und ausgezogenem Aufnahme- korb,
- Figur 2: eine perspektivische Darstellung eines Aufnahmekorbs mit einem Anschlussadapter und mit einem eingelegten Endoskop,
- Figur 3: einen senkrechten Querschnitt durch das Gerät gemäss Figur 1,
- Figur 4: einen horizontalen Querschnitt durch das Gerät gemäss Figur 3 über dem Aufnahmekorb,
- Figur 5: eine Seitenansicht auf die im Gerät gemäss Figur 3 eingebaute Messeinheit mit mehreren Kanalsystemen,
- Figur 6: eine Draufsicht auf die Messeinheit gemäss Figur 5,
- Figur 7: einen senkrechten Querschnitt durch die Messeinheit gemäss Figur 5,
- Figur 8: eine perspektivische Darstellung von schräg oben auf die Messeinheit gemäss Figur 5,
- Figur 9: einen Teilquerschnitt durch die Messeinheit gemäss Figur 8,
- Figur 10: einen vergrösserter Querschnitt auf eine einzelne Messzelle der Messeinheit gemäss Figur 9,
- Figur 11: ein Temperatur/Zeit-Diagramm eines Aufbereitungs- prozesses mit verschiedenen Behandlungsfluiden,
- Figur 12: einen alternativen Aufnahmekorb für das Gerät gemäss Figur 1, jedoch mit zwei Aufnahmeebenen für Geräte,
- Figur 13: eine perspektivische Darstellung auf einen Anschluss- adapter für zwei Behandlungsebenen,
- Figur 14: den in Figur 2 gezeigten Anschlussadapter in etwas vergrösserter Darstellung, und
- Figur 15: ein schematisches Beispiel eines Endoskops mit mehre- ren Kanälen.

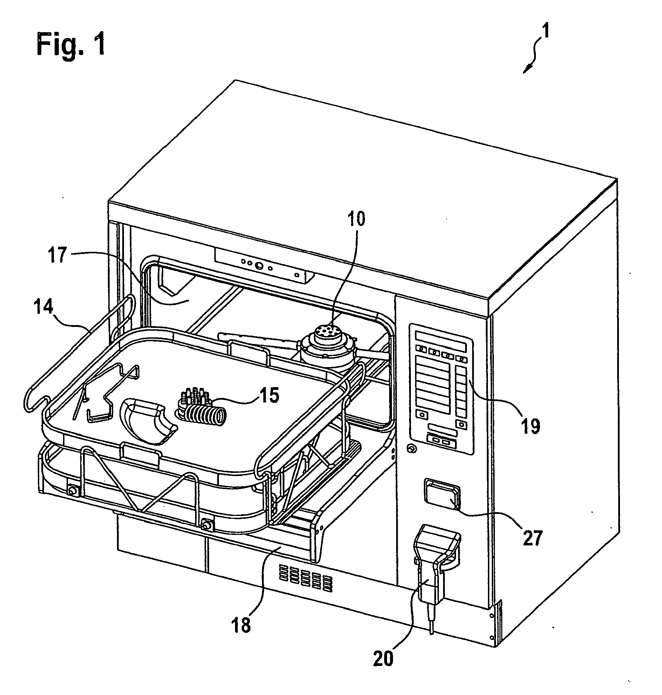

Wie in Figur 1 dargestellt, verfügt ein allgemein mit 1 bezeichnetes Aufbereitungsgerät über eine Waschkammer 17, die mittels einer Waschkammertüre 18 verschliessbar ist. Ein Aufnahmekorb 14 für die zu behandelnden Endoskope kann über geeignete Führungen in die Waschkammer 17 eingeschoben werden. Ein am Boden der Waschkammer gerade noch sichtbarer Anschlusskopf 10 kommuniziert bei eingeschobenem Aufnahmekorb 14 und bei geschlossener Waschkammertüre 18 mit einem Anschlussadapter 15 am Boden des Aufnahmekorbs auf nachstehend noch beschriebene Art und Weise. Das Gerät verfügt über ein Bedienungspanel 19, an dem die einzelnen Prozesse eingestellt werden können bzw. an dem die jeweiligen Prozessschritte ablesbar sind. Am Gerät ist ausserdem ein Barcodeleser 20 angeordnet, mit dessen Hilfe die kodierten Endoskope vor dem Einschieben in die Waschkammer identifiziert werden können. Auf diese Weise können jedem Endoskop die spezifischen Aufbereitungsschritte zugeordnet werden, wobei ein Drucker 27 ein Chargenprotokoll ausdrucken kann.

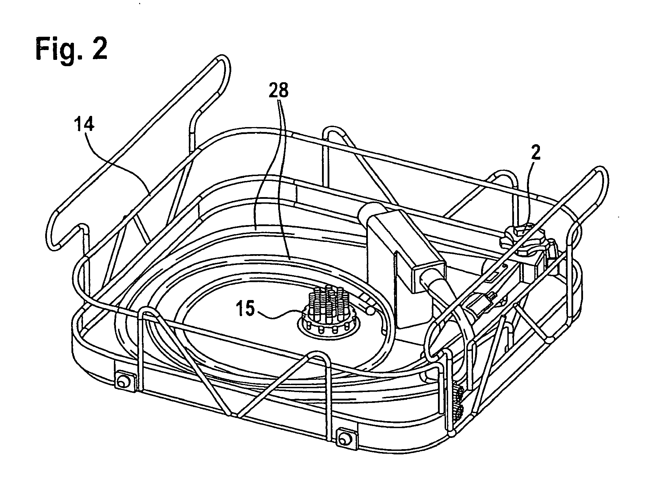

In Figur 2 ist dargestellt, wie ein Endoskop 2 mit seinen Schlauchleitungen 28 im Aufnahmekorb 14 angeordnet ist. Der Aufnahmekorb 14 besteht beim Ausführungsbeispiel aus einer Drahtkonstruktion, könnte aber auch eine beliebige andere Bauweise aufweisen. Am Boden des Aufnahmekorbs ist der bereits erwähnte Anschlussadapter 15 angeordnet, wobei die einzelnen Schlauchleitungen 28 des Endoskops aber noch nicht auf die einzelnen Adapteranschlüsse aufgesteckt sind.

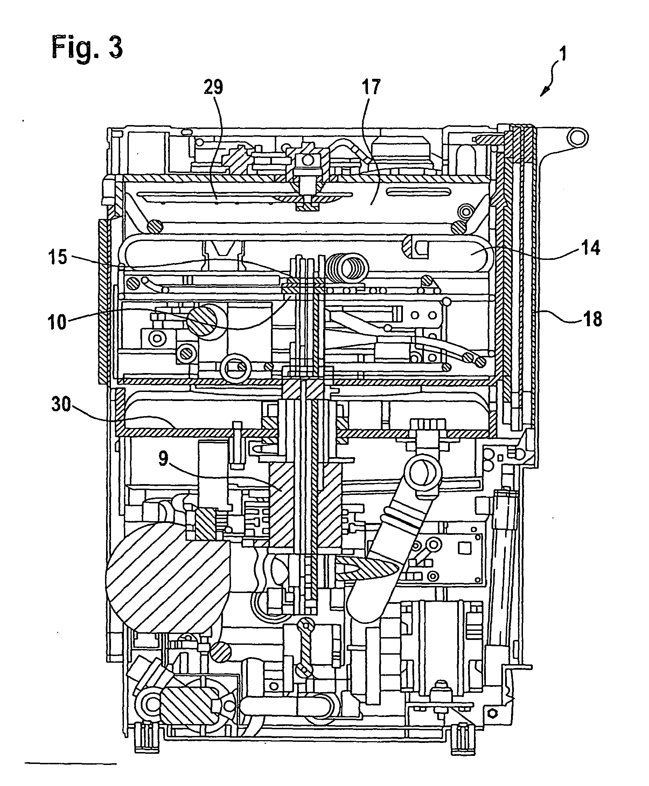

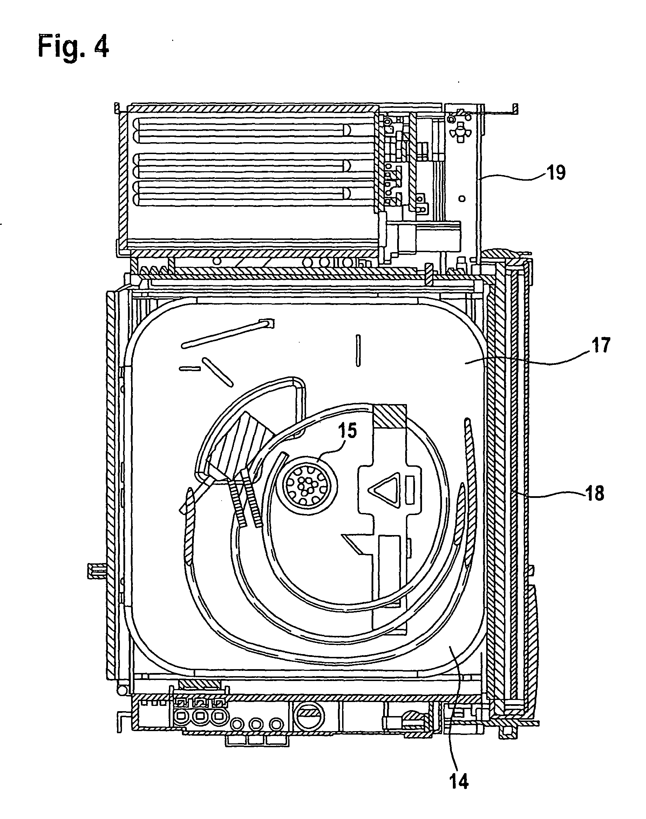

Weitere konstruktive Einzelheiten des Geräts sind aus den Figuren 3 und 4 ersichtlich. Die Waschkammertüre 18 ist hier geschlossen und der Aufnahmekorb 14 in seine Betriebsstellung eingeschoben. Wie dargestellt, befindet sich der Anschlusskopf 10 jetzt in der angehobenen Betriebsstellung, in welcher er von unten an den Anschlussadapter 15 des Aufnahmekorbs 14 angedockt ist. Der Anschlusskopf 10 ist Bestandteil einer insgesamt mit 9 bezeichneten Messeinheit, die am Boden 30 der Waschkammer angeordnet ist und von welcher der grössere Teil unterhalb dieses Bodens liegt. Das Aufbereitungsgerät verfügt selbstverständlich noch über weitere, hier nicht näher beschriebene Komponenten, wie z.B. einen im oberen Bereich der Waschkammer 17 angeordneten Sprüharm 29 für die äussere Behandlung der im Aufnahmekorb 14 liegenden Endoskope. Auch Förderpumpen, Heizeinrichtungen und Tanks für Behandlungszusätze sind im Gerät angeordnet.

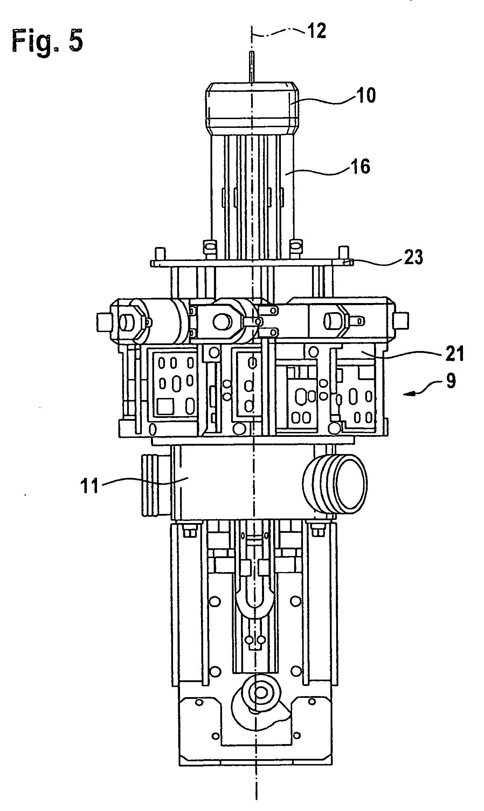

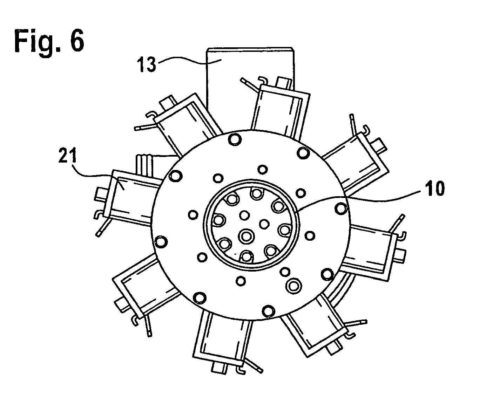

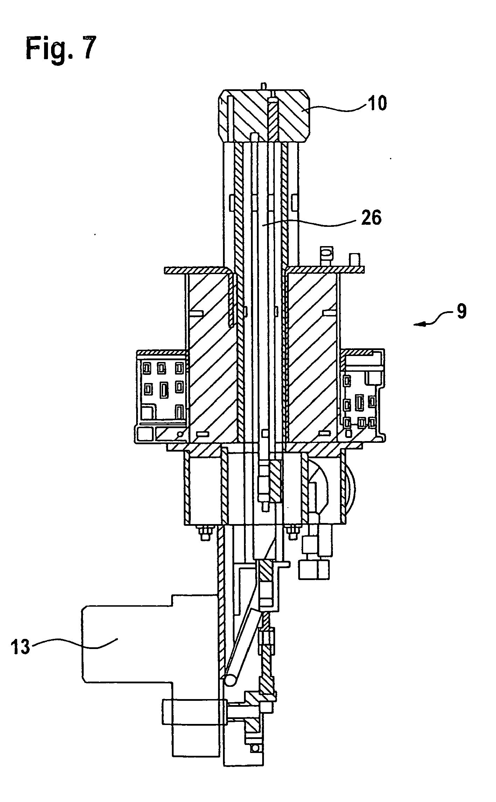

Die Figuren 5 bis 7 zeigen ein paar weitere Einzelheiten der Messeinheit 9. Die Befestigung der Messeinheit am Boden der Waschkammer erfolgt mit Hilfe eines Befestigungsflansches 23. Die ganze Messeinheit besteht aus insgesamt acht einzelnen Messzellen 21, von denen jede ein eigenes Kanalsystem bildet und die kreisförmig in regelmässiger Teilung um eine zentrale Achse 12 angeordnet sind. Aus dem kreisringförmigen Flansch 23 ragen parallele Steigrohre 16, die durch den Anschlusskopf 10 abgeschlossen sind. Die Hubbewegung zum Andocken des Anschlusskopfs 10 an den jeweiligen Anschlussadapter erfolgt mit Hilfe einer im unteren Bereich angeordneten Hubvorrichtung 13. Die zentrale Versorgung sämtlicher Kanalsysteme erfolgt über eine gemeinsame Verteilkammer 11, die unter den Messzellen 21 angeordnet ist.

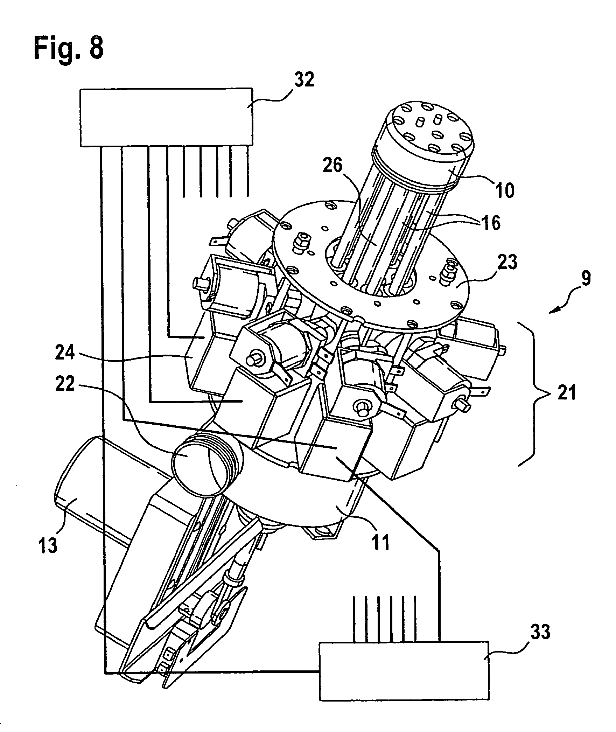

Weitere Details der Messeinheit und des durch sie gebildeten Kanalsystems bzw. einer einzelnen Messzelle ergeben sich aus den Figuren 8 bis 10. Aus Figur 8 ist nochmals die Gesamtanordnung der einzelnen Messzellen 21 der Messeinheit 9 sichtbar. Die Behandlungsfluide, nämlich Luft und Wasser werden über einen Anschlussstutzen 22 der Verteilkammer 11 zugeführt. Die Bewegung des Anschlusskopfs 10 mit den Steigrohren 16 erfolgt über eine zentrale Hubspindel 26, die über ein Getriebe mit der Hubvorrichtung 13 verbunden ist.

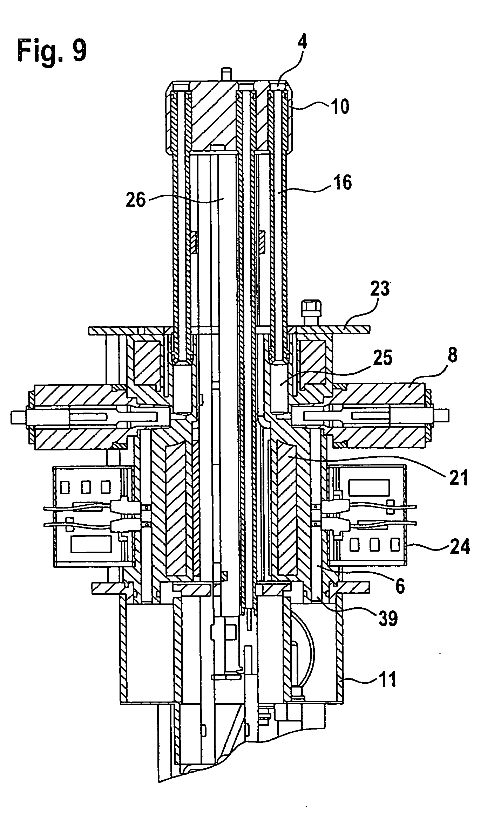

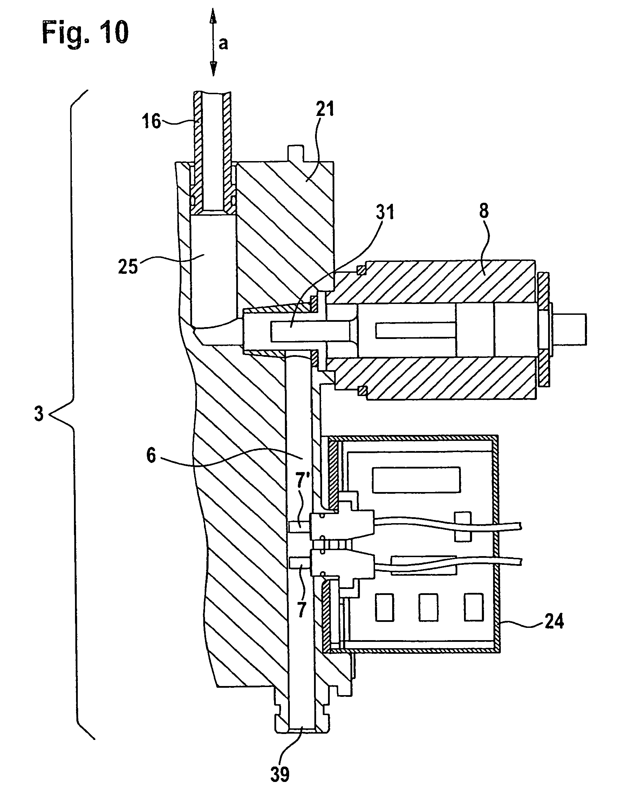

Wie insbesondere aus Figur 9 ersichtlich ist, bewegt sich jedes einzelne Steigrohr 16 teleskopartig in einem Teleskopführungsrohr 25, wobei es über geeignete Dichtmittel wie z.B. O-ringe, abgedichtet ist. Die Steigrohre 16 münden am Anschlusskopf 10 jeweils in einen Kanalanschluss 4, der das Ende eines Leitungssystems bildet. Dieses Leitungssystem beginnt an der Verteilkammer 11 bzw. an einem Fluidanschluss 39 mit einer Messstrecke 6 einer Messzelle 21 und verläuft dann an einem Einlassventil 8 vorbei über das Teleskopführungsrohr 25 zum Steigrohr 16. Einzelheiten einer Messzelle 21 sind in Figur 10 dargestellt.

In der Messstrecke 6 sind zwei thermoelektrische Sensoren 7 und 7' unmittelbar nacheinander angeordnet. Die Sensoren sind in einem Sensorgehäuse 24 fixiert, das auch die erforderliche Auswertelektronik für eine redundante Signalmessung enthält. Das obere Ende der Messstrecke 6 mündet in einen Ventilsitz 31, der einem Einlassventil 8 zugeordnet ist. Dieses Einlassventil 8 ist unmittelbar über dem Sensorgehäuse 24 angeordnet, wie auch aus Figur 8 ersichtlich ist. Vom Ventilsitz 31 führt das Leitungssystem zum Teleskopführungsrohr 25, das wie die Messstrecke 6 als Bohrung in einem Materialblock ausgebildet ist. Im Teleskopführungsrohr 25 ist das Steigrohr 16 in Pfeilrichtung a verschiebbar.

Bei einem Aufbereitungsvorgang wird der bestückte Aufnahmekorb in die Waschkammer eingefahren und die Waschkammertüre wird geschlossen. Jeder Einzelkanal des Endoskops ist dabei separat an einem der Stutzen des Anschlussadapters 15 angeschlossen. Sensoren überprüfen die korrekte Schliessstellung der Waschkammertüre, worauf der Prozess gestartet werden kann. Dabei wird zuerst der Anschlusskopf 10 von unten an den Anschlussadapter 15 angedockt. Ist diese Betriebsstellung erreicht, beginnt die Aufbereitung des Endoskops einerseits indem verschiedene Behandlungsfluide durch die Kanäle durchgeleitet werden und andererseits auch durch äusserliche Behandlung mit Behandlungsfluiden. Eine in Figur 8 allgemein mit 32 bezeichnete Steuerung vergleicht den in der jeweiligen Messstrecke 6 gemessenen Durchfluss mit einem in der Steuerung abgespeicherten Nominaldurchfluss für das zu behandelnde Endoskop. Bei einer Abweichung wird ein Warnsignal generiert, das aufgezeichnet wird und gegebenenfalls den Prozess unterbricht. Dadurch können auch versehentlich nicht angeschlossene Endoskopkanäle oder vollständig blockierte Endoskopkanäle rechtzeitig erkannt werden. Selbstverständlich ist die Steuerung mit jeder einzelnen der insgesamt acht Messzellen 21 verbunden.

Die Steuerung überträgt den in den einzelnen Messstrecken 6 gemessenen Durchfluss an eine unabhängige Messdatenerfassung 33. Diese vergleicht den von der Steuerung 32 erfassten Durchfluss mit dem entsprechenden Durchfluss über den zweiten, redundanten Sensor 7' in derselben Messstrecke 6. Bei Abweichung der Durchflüsse wird ebenfalls eine Warnmeldung generiert.

Das gesamte Messsystem ist verträglich zu Waschlauge, Desinfektionsmittel, normalem oder entsalztem Wasser und Luft. Das Messsystem kann somit den gleichen Aufbereitungsprozess durchlaufen wir die zu behandelnden Instrumente selber. Ausserdem kann das Messsystem Temperaturen bis zu 93° C. ausgesetzt werden, womit eine thermische Desinfektion möglich ist. Dieser thermische Desinfektionsprozess kann in regelmässigen Intervallen, beispielsweise alle zwölf Stunden stattfinden.

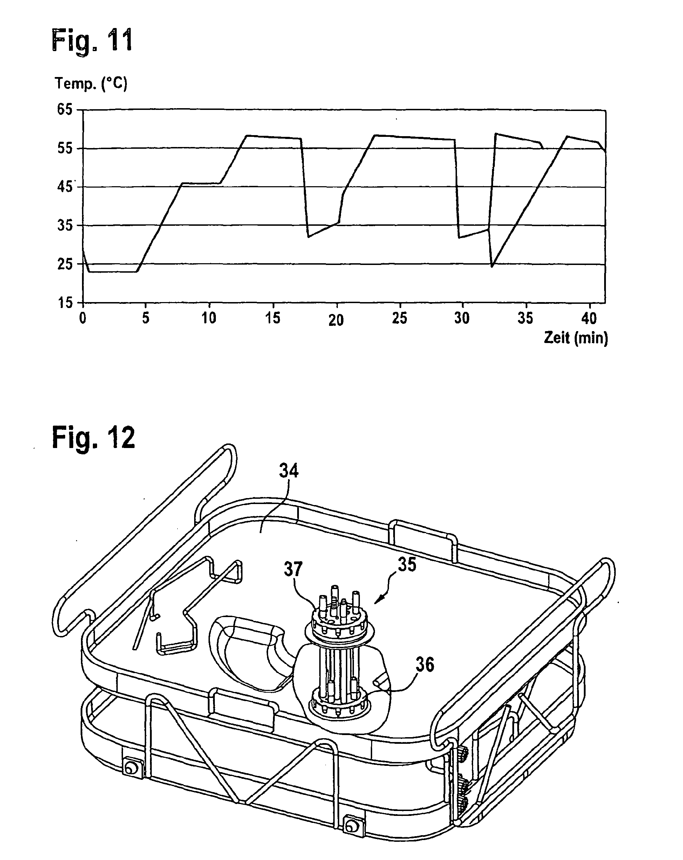

Im Diagramm gemäss Figur 11 ist ein Aufbereitungsprozess dargestellt, der etwas mehr als 40 Minuten dauert. Während der ersten fünf Minuten findet ein kaltes Vorwaschen und ein dosiertes Zuführen von Reinigungsmitteln statt. Anschliessend wird bis auf ca. 45° C aufgeheizt und ca. drei Minuten gewaschen. Danach wird bis ca. 58° C aufgeheizt und nochmals ca. drei Minuten gewaschen. Danach erfolgt ein Abpumpen und ein Kaltwassereinlauf mit einer einminütigen Zwischenspülung bei rund 35° C Das Zwischenspülmittel wird sodann abgepumpt und es erfolgt nochmals eine dosierte Zufuhr von Desinfektionsmittel. Es wird wiederum während ca. fünf Minuten bei 58° C desinfiziert, danach abgepumpt und ca. eine Minute zwischengespült. Nach insgesamt ca. 32 Minuten Prozesszeit erfolgt wieder ein Aufheizen und Spülen bei 58° C während ca. 1,5 Minuten und danach wird abgepumpt, kondensiert und getrocknet.

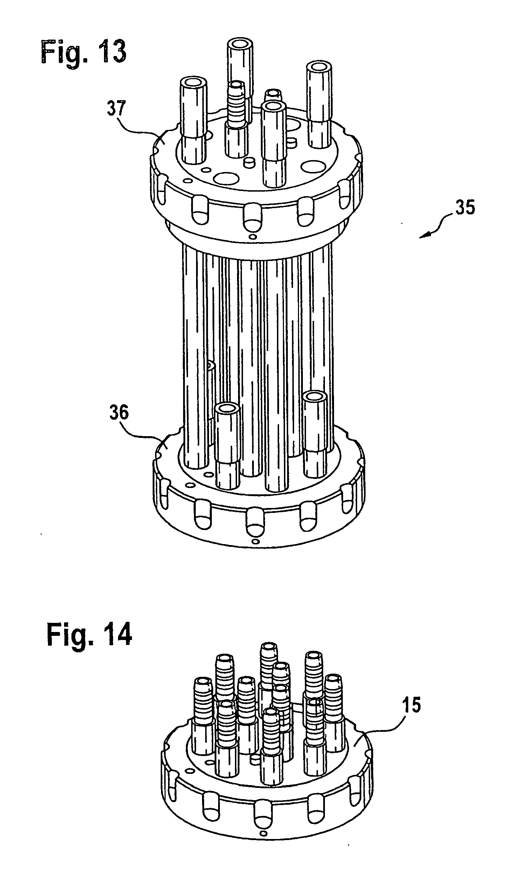

Figur 12 zeigt eine alternative Ausführung eines Doppelstock-Aufnahmekorbs 34. In diesem können auf zwei verschiedenen Etagen Endoskope gelagert werden, wobei ein Doppelanschluss-Adapter 35 zu beiden Etagen führt. Dieser ist etwas vergrössert auch in Figur 13 dargestellt. Der Doppelanschluss-Adapter verfügt über einen oberen Anschluss 35 und einen unteren Anschluss 36, der in Figur 12 in Wirklichkeit nicht sichtbar wäre. Im Vergleich dazu ist in Figur 14 nochmals der Anschlussadapter 15 für nur eine Ebene dargestellt. Ersichtlicherweise verfügt der einfache Anschlussadapter 15 über insgesamt acht Kanalanschlüsse im Aussenbereich und zusätzlich drei Hilfsanschlüsse im Zentrum, der Doppelanschluss-Adapter 35 jedoch an jedem Anschluss nur über vier Kanalanschlüsse und zusätzliche Hilfsanschlüsse. Die Hilfsanschlüsse dienen der Trocknung und der Leckluftprüfung. Mit dem einfachen Anschlussadapter 15 kann denn auch ein Endoskop mit maximal acht Einzelkanälen aufbereitet werden, während mit dem Doppelanschluss-Adapter 35 auf jeder Etage nur ein Endoskop mit maximal vier Einzelkanälen behandelbar ist.

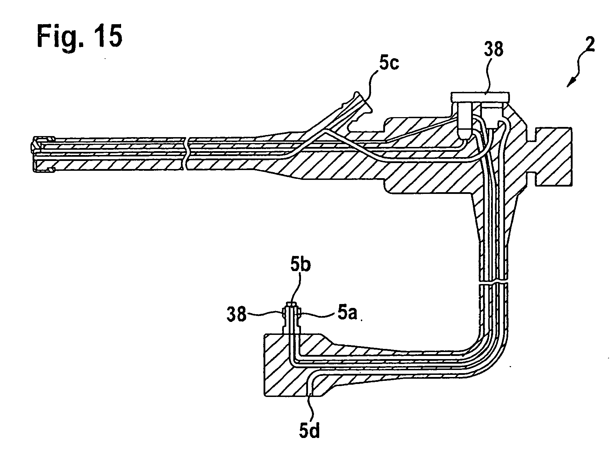

In Figur 15 ist schematisch ein Endoskop 2 dargestellt, an dem vier separate Kanäle einzeln durchspült werden können. Es können demnach zwei derartige Endoskope mit einem Gerät mit insgesamt acht Kanalsystemen behandelt werden. Die am Anschlussadapter anzuschliessenden Anschlüsse sind folgende: 5a für den Wasserkanal, 5b für den Luftkanal, 5c für den Biopsiekanal und 5d für den Saugkanal. Speziell für die Endoskope geschaffene Kanaltrenner 38 sorgen dafür, dass die Luft- und Wasserkanäle bei der Behandlung voneinander getrennt bleiben. Jeder einzelne der vier Kanäle durchläuft bei der Behandlung genau den gleichen Prozess bezüglich Behandlungsfluid, Temperatur und Durchflussmessung.

Selbstverständlich können die erfindungsgemässen Geräte an die spezifischen Instrumente angepasst werden, die zu behandeln sind. Dies betrifft insbesondere die Anzahl und konstruktive Anordnung der Kanalsysteme.

## Patentansprüche

1. Vorrichtung zum Behandeln und Analysieren von Kanälen in Instrumenten, insbesondere in Endoskopen
mit
- einem Leitungssystem (3) zum Zuführen wenigstens eines Behandlungsfluids zu einem Kanalanschluss (4), an dem ein Kanal des Instruments anschliessbar ist,
- wobei das Leitungssystem einen Fluidanschluss (39) aufweist, der mit einer Behandlungsfluid-Quelle verbindbar ist,
- sowie mit einer Messeinrichtung zum Messen der Strömung des Behandlungsfluids im Leitungssystem (3) und damit der Durchlässigkeit des angeschlossenen Kanals, wobei
- das Leitungssystem (3) eine Messstrecke (6) aufweist,
- und die Messeinrichtung über wenigstens einen Strömungssensor (7, 7') verfügt, der in der Messstrecke angeordnet ist,
- wobei alle Behandlungsschritte über das Leitungssystem und insbesondere über die Messstrecke mit dem Strömungssensor ausführbar sind, **dadurch gekennzeichnet, dass** in der Messstrecke (6) zwei Strömungssensoren (7,7') nacheinander angeordnet sind, mit denen die Strömung redundant messbar ist.

2. Vorrichtung nach Anspruch 1, vorzugsweise mit Fördermittel zum Durchleiten des Behandlungsfluids durch das Leitungssystem und durch den angeschlossenen Kanal, **dadurch gekennzeichnet, dass** die Messstrecke (6) bezogen auf eine horizontale Ebene unterhalb der Ebene verläuft, auf der der Kanalanschluss (4) liegt, wobei das Leitungssystem (3) vorzugsweise derart zur horizontalen Ebene geneigt ist, dass es bei deaktiviertem Fördermittel von einer Flüssigkeit entleerbar ist.

3. Vorrichtung nach Anspruch 1 oder 2, **dadurch gekennzeichnet, dass** der Strömungssensor (7, 7') ein thermoelektrischer Sensor ist.

4. Vorrichtung nach einem der Ansprüche 1 bis 3, **dadurch gekennzeichnet, dass** zwischen dem Strömungssensor (7, 7') und dem Kanalanschluss (4) ein Einlassventil (8) angeordnet ist.

5. Vorrichtung nach einem der Ansprüche 1 bis 4, **dadurch gekennzeichnet, dass** mehrere Leitungssysteme (3) zu einer Messeinheit (9) zusammengefasst sind, wobei jedes Leitungssystem zu einem Kanalanschluss (4) führt und wobei vorzugsweise alle Kanalanschlüsse (4) auf einem gemeinsamen Anschlusskopf (10) angeordnet sind.

6. Vorrichtung nach Anspruch 5, **dadurch gekennzeichnet, dass** alle Leitungssysteme über eine gemeinsame Verteilkammer (11) mit dem Behandlungsfluid speisbar sind.

7. Vorrichtung nach Anspruch 5 oder 6, **dadurch gekennzeichnet, dass** alle Leitungssysteme, vorzugsweise wenigstens vier, vorzugsweise kreisförmig oder sternförmig um eine zentrale, vorzugsweise vertikale Achse angeordnet sind.

8. Vorrichtung nach Anspruch 5 und Anspruch 7, **dadurch gekennzeichnet, dass** der Anschlusskopf (10) mittels einer Hubvorrichtung (13) aus einer Ruhestellung von der Messeinheit (9) weg in eine vorzugsweise höher gelegene Betriebsstellung anhebbar ist, in der die Leitungsanschlüsse an einen, einem Aufnahmekorb (14) für die Instrumente zugeordneten Anschlussadapter (15) andockbar sind.

9. Vorrichtung nach Anspruch 8, **dadurch gekennzeichnet, dass** die Verbindung zwischen der Messeinheit und dem Anschlusskopf über Steigrohre (16) erfolgt, die teleskopartig in der Messeinheit verschiebbar sind.

10. Vorrichtung nach einem der Ansprüche 1 bis 9, **dadurch gekennzeichnet, dass** das Leitungssystem (3) ausschliesslich starre Rohrleitungen bzw. Leitungen aufweist.

11. Verfahren zum Behandeln und Analysieren von Kanälen in Instrumenten, insbesondere in Endoskopen bei dem
- in einem Leitungssystem (3) wenigstens ein Behandlungsfluid einem Kanalanschluss (4) zugeführt wird, an dem ein Kanal des Instruments angeschlossen ist,
- wobei mittels einer Messeinrichtung die Strömung des Behandlungsfluids im Leitungssystem (3) und damit die Durchlässigkeit des angeschlossenen Kanals gemessen wird, und wobei
- die Messung mittels eines Strömungssensors (7, 7') an einer Messstrecke (6) erfolgt,
- und alle Behandlungsschritte über das Leitungssystem und insbesondere über die Messstrecke mit dem Strömungssensor ausgeführt werden, **dadurch gekennzeichnet, dass** die Stömung an jeder Messtrecke (6) mitels zwei Strömungssensoren (7,7') redundant gemessen wird und dass die beiden Messsungen unabhängig ausgewertet und miteinander verglichen werden.

12. Verfahren nach Anspruch 11, **dadurch gekennzeichnet, dass** das Leitungssystem den gleichen Behandlungsschritten ausgesetzt wird wie das Instrument, insbesondere auch den gleichen Behandlungstemperaturen und insbesondere auch einer chemischen und/oder thermischen Desinfektion.

13. Verfahren nach Anspruch 11 oder 12, **dadurch gekennzeichnet, dass** die Messung an einer Messstrecke (6) erfolgt, in der das Behandlungsfluid bezogen auf eine horizontale Ebene von unten nach oben gefördert wird.

14. Verfahren nach einen der Ansprüche 11 bis 13, **dadurch gekennzeichnet, dass** das Behandlungsfluid gleichzeitig unter dem gleichen Druck durch mehrere Leitungssysteme (3) gefördert wird, von denen jedes zu einem Kanalanschluss (4) führt und dass die Strömung an jedem Leitungssystem (3) gemessen wird.

15. Verfahren nach einen der Ansprüche 11 bis 14, **dadurch gekennzeichnet, dass** verschiedene Behandlungsfluide nacheinander durch das gleiche Leitungssystem (3) gefördert werden und dass die Strömung einzelner oder aller Behandlungsfluide gemessen wird.

## Claims

1. Device for treating and analysing channels in instruments, particularly in endoscopes
with
- a conduit system (3) for feeding at least one treatment fluid to a channel attachment (4), to which a channel of the instrument can be attached,
- wherein the conduit system has a fluid attachment (39), which can be connected to a source of treatment fluid,
- and with a measuring device for measuring the flow of the treatment fluid in the conduit system (3), and thus the permeability of the attached channel, wherein
- the conduit system (3) has a measurement section (6),
- and the measuring device has at least one flow sensor (7, 7'), which is arranged in the measurement section,
- wherein all the treatment steps can be carried out via the conduit system and in particular via the measurement section with the flow sensor,
**characterized in that** two flow sensors (7, 7'), with which the flow can be measured redundantly, are arranged one after the other in the measurement section (6).

2. Device according to Claim 1, preferably with delivery means for conveying the treatment fluid through the conduit system and through the attached channel, **characterized in that** the measurement section (6), in relation to a horizontal plane, extends underneath the plane on which the channel attachment (4) lies, wherein the conduit system (3) is preferably inclined with respect to the horizontal plane in such a way that it can be emptied of a liquid when the delivery means is deactivated.

3. Device according to Claim 1 or 2, **characterized in that** the flow sensor (7, 7') is a thermoelectric sensor.

4. Device according to one of Claims 1 to 3, **characterized in that** an inlet valve (8) is arranged between the flow sensor (7, 7') and the channel attachment (4).

5. Device according to one of Claims 1 to 4, **characterized in that** several conduit systems (3) are combined to form a measuring unit (9), wherein each conduit system leads to a channel attachment (4), and wherein preferably all the channel attachments (4) are arranged on a common attachment head (10).

6. Device according to Claim 5, **characterized in that** all the conduit systems can be fed with the treatment fluid via a common distributor chamber (11).

7. Device according to Claim 5 or 6, **characterized in that** all the conduit systems, preferably at least four, are preferably arranged in a circle shape or star shape around a central, preferably vertical axis.

8. Device according to Claim 5 and Claim 7, **characterized in that** the attachment head (10) can be lifted away from the measuring unit (9), by means of a lifting device (13), from a rest position to a preferably higher operating position, in which the conduit attachments can be docked onto an attachment adapter (15) assigned to a receiving basket (14) for the instruments.

9. Device according to Claim 8 **characterized in that** the connection between the measuring unit and the attachment head is effected via riser pipes (16), which are movable telescopically in the measuring unit.

10. Device according to one of Claims 1 to 9, **characterized in that** the conduit system (3) exclusively comprises rigid tubular conduits or lines.

11. Method for treating and analysing channels in instruments, particularly in endoscopes, in which
- in a conduit system (3), at least one treatment fluid is fed to a channel attachment (4), to which a channel of the instrument is attached,
- wherein the flow of the treatment fluid in the conduit system (3), and thus the permeability of the attached channel, is measured by means of a measuring device, and wherein
- the measurement is effected by means of a flow sensor (7, 7') on a measurement section (6),
- and all the treatment steps are carried out via the conduit system and in particular via the measurement section with the flow sensor,
**characterized in that** the flow on each measurement section (6) is measured redundantly by means of two flow sensors (7, 7'), and **in that** the two measurements are evaluated independently and compared to each other.

12. Method according to Claim 11, **characterized in that** the conduit system is subjected to the same treatment steps as the instrument, in particular also the same treatment temperatures and in particular also a chemical and/or thermal disinfection.

13. Method according to Claim 11 or 12, **characterized in that** the measurement is effected on a measurement section (6) in which the treatment fluid is delivered from the bottom upwards in relation to a horizontal plane.

14. Method according to one of Claims 11 to 13, **characterized in that** the treatment fluid is delivered at the same pressure simultaneously through several conduit systems (3), each of which leads to a channel attachment (4), and **in that** the flow is measured on each conduit system (3).

15. Method according to one of Claims 11 to 14, **characterized in that** different treatment fluids are delivered one after another through the same conduit system (3), and **in that** the flow of individual treatment fluids or of all the treatment fluids is measured.

## Revendications

1. Dispositif de traitement et d'analyse de canaux dans des instruments, en particulier des endoscopes, avec
- un réseau de conduites (3) pour amener au moins un fluide de traitement à un raccord de canal (4), auquel un canal de l'instrument peut être raccordé,
- dans lequel le réseau de conduites présente un raccord de fluide (39), qui peut être raccordé à une source de fluide de traitement,
- ainsi qu'avec un dispositif de mesure pour mesurer l'écoulement du fluide de traitement dans le réseau de conduites (3) et de ce fait la perméabilité du canal raccordé, dans lequel
- le réseau de conduites (3) présente une zone de mesure (6),
- et le dispositif de mesure est muni d'au moins un détecteur d'écoulement (7, 7'), qui est disposé dans la zone de mesure,
- dans lequel toutes les étapes de traitement peuvent être exécutées sur le réseau de conduites et en particulier sur la zone de mesure avec le détecteur d'écoulement,
**caractérisé en ce qu'**il se trouve dans la zone de mesure (6) deux détecteurs d'écoulement (7, 7') disposés l'un derrière l'autre, avec lesquels l'écoulement peut être mesuré de manière redondante.

2. Dispositif selon la revendication 1, de préférence avec un moyen de transport pour acheminer le fluide de traitement à travers le réseau de conduites et à travers le canal raccordé, **caractérisé en ce que** la zone de mesure (6) s'étend, par rapport à un plan horizontal, en dessous du plan sur lequel se situe le raccord de canal (4), dans lequel le réseau de conduites (3) est incliné par rapport au plan horizontal, de préférence de telle manière qu'il puisse être vidé d'un liquide lorsque le moyen de transport est désactivé.

3. Dispositif selon la revendication 1 ou 2, **caractérisé en ce que** le détecteur d'écoulement (7, 7') est un détecteur thermoélectrique.

4. Dispositif selon l'une quelconque des revendications 1 à 3, **caractérisé en ce qu'**une soupape d'admission (8) est disposée entre le détecteur d'écoulement (7, 7') et le raccord de canal (4).

5. Dispositif selon l'une quelconque des revendications 1 à 4, **caractérisé en ce que** plusieurs réseaux de conduites (3) sont rassemblés en une unité de mesure (9), dans lequel chaque réseau de conduites conduit à un raccord de canal (4) et dans lequel de préférence tous les raccords de canaux (4) sont disposés sur une tête de raccordement commune (10).

6. Dispositif selon la revendication 5, **caractérisé en ce que** tous les réseaux de conduites (3) peuvent être alimentés en fluide de traitement via une chambre de répartition commune (11).

7. Dispositif selon la revendication 5 ou 6, **caractérisé en ce que** tous les réseaux de conduite, de préférence au moins quatre, sont disposés de préférence en forme de cercle ou en forme d'étoile autour d'un axe central, de préférence vertical.

8. Dispositif selon la revendication 5 et la revendication 7, **caractérisé en ce que** la tête de raccordement (10) peut être relevée, au moyen d'un dispositif de levage (13), d'une position de repos à l'écart de l'unité de mesure (9) à une position de fonctionnement située de préférence plus haut, dans laquelle les raccords de conduites peuvent être couplés à un adaptateur de raccordement (15) associé à un panier de réception (14) pour les instruments.

9. Dispositif selon la revendication 8, **caractérisé en ce que** la liaison entre l'unité de mesure et la tête de raccordement est réalisée par des conduites montants (16), qui peuvent coulisser de manière télescopique dans l'unité de mesure.

10. Dispositif selon l'une quelconque des revendications 1 à 9, **caractérisé en ce que** le réseau de conduites (3) présente exclusivement des tubes ou des conduites rigides.

11. Procédé de traitement et d'analyse de canaux dans des instruments, en particulier dans des endoscopes, dans lequel
- dans un réseau de conduites (3), on fournit au moins un fluide de traitement à un raccord de canal (4), auquel un canal de l'instrument est raccordé,
- dans lequel on mesure, au moyen d'un dispositif de mesure, l'écoulement du fluide de traitement dans le réseau de conduites (3), et de ce fait la perméabilité du canal raccordé, et dans lequel
- on effectue la mesure sur une zone de mesure (6) au moyen d'un détecteur d'écoulement (7, 7'),
- et on effectue toutes les étapes de traitement sur le réseau de conduites et en particulier sur la zone de mesure avec le détecteur d'écoulement,
**caractérisé en ce que** l'on mesure de manière redondante l'écoulement dans chaque zone de mesure (6) au moyen de deux détecteurs d'écoulement (7, 7') et on analyse les deux mesures de manière indépendante et on les compare l'une à l'autre.

12. Procédé selon la revendication 11, **caractérisé en ce que** l'on soumet le réseau de conduites aux mêmes étapes de traitement que l'instrument, en particulier aussi aux mêmes températures de traitement et en particulier aussi à une désinfection chimique et/ou thermique.

13. Procédé selon la revendication 11 ou 12, **caractérisé en ce que** l'on effectue la mesure dans une zone de mesure (6), dans laquelle le fluide de traitement est transporté de bas en haut par rapport à un plan horizontal.

14. Procédé selon l'une quelconque des revendications 11 à 13, **caractérisé en ce que** l'on transporte le fluide de traitement simultanément sous la même pression à travers plusieurs réseaux de conduites (3), dont chacun conduit à un raccord de canal (4) et **en ce que** l'on mesure l'écoulement dans chaque réseau de conduites (3).

15. Procédé selon l'une quelconque des revendications 11 à 14, **caractérisé en ce que** l'on transporte différents fluides de traitement l'un après l'autre à travers le même réseau de conduites (3) et **en ce que** l'on mesure l'écoulement des fluides de traitement individuellement ou tous ensemble.
